# EUROPÄISCHE PATENTSCHRIFT

(11) **EP 1 256 567 B1**
(45) Veröffentlichungstag und Bekanntmachung des Hinweises auf die Patenterteilung: **25.08.2004**
(21) Anmeldenummer: 02009220.1
(22) Anmeldetag: 25.04.2002
(51) Int. Cl.: C07C 209/10, C07C 263/10, C07C 211/58, C07C 265/14

(54) **Verfahren zur Herstellung von 1,4-Diaminonaphthalin und/oder 1,5-Diaminonaphthalin**
Process for the preparation of 1,4-diaminonaphthalene and/or 1,5-diaminonaphthalene
Procédé pour la préparation du 1,4-diamine de naphtalène et/ou du 1,5-diamine de naphtalène

(30) Priorität: 08.05.2001 DE 10122234
(43) Veröffentlichungstag der Anmeldung: 13.11.2002
(73) Patentinhaber: Bayer MaterialScience AG, 51368 Leverkusen (DE)
(72) Erfinder: Zechlin, Joachim, Dr., 41472 Neuss (DE); Duda, Lothar, Dr., 40227 Düsseldorf (DE); Wegener, Gerhard, Dr., 40822 Mettmann (DE); Hoffmann, Andreas, Dr., 50733 Köln (DE); Temme, Bodo, Dr., League City, Texas 77573 (US)

(56) Entgegenhaltungen:
- DE-C- 45 549
- DE-C- 234 912
- US-A- 1 775 360
- US-A- 2 391 848
- US-A- 4 405 527
- SALKIND, J.; STETZURO, Z.: "Umlagerungen der Dibromnaphthaline durch Aluminiumchlorid" CHEMISCHE BERICHTE, Bd. 64, - 1931 Seite 953-954 XP001097475

## Beschreibung

Die vorliegende Erfindung betrifft ein Verfahren zur Herstellung von 1,4-Diaminonaphthalin und/oder 1,5-Diaminonaphthalin ausgehend von Naphthalin.

1,4-Diisocyanatonaphthalin wurde als Ausgangsstoff für Polyurethane in der Literatur erstmals erwähnt in Ann. Chem. 562, 6 (1949). Durch das Fehlen eines kostengünstigen Herstellungsverfahrens zum 1,4-Diaminonaphthalin und somit zum 1,4-Diisocyanatonaphthalin sind in der Literatur nur wenige Einsatzgebiete für 1,4-Diisocyanatonaphthalin beschrieben. Beispiel für solche Einsatzgebiete sind die Herstellung von Polyurethan-basierten Klebstoffen oder Faser-Additiven, die in JP-A2-52096295 und DE-A1-2403656 beschrieben sind.

Als Isocyanatkomponente für die Herstellung von Polyurethanen auf Naphthalinbasis wird nach dem Stand der Technik in erster Linie 1,5-Diisocyanatonaphthalin eingesetzt, das bei der Herstellung von Polyurethan-Elastomeren Verwendung findet.

Die Herstellung von 1,5-Diisocyanatonaphthalin erfolgt industriell durch die Phosgenierung des entsprechenden Diamins 1,5-Diaminonaphthalin. Nach dem Stand der Technik wird 1,5-Diaminonaphthalin über die Sulfonierung von Naphthalin und den anschließenden Austausch der Sulfonsäuregruppen gegen Aminogruppen hergestellt (DE-C1-3840618).

1,4-Diaminonaphthalin dagegen wird bisher lediglich neben der Umsetzung zum 1,4-Napthalindiisocyanat für die Synthese von Polyamiden, Polyimiden und Polyiminen zur Herstellung von leitfähigen oder elektrolumineszenten Polymer-Materialien eingesetzt, wie dies in den Patentschriften JP-A2-06346050 und JP-A2-07022670 beispielhaft dargestellt ist.

Für die Herstellung von 1,4-Diaminonaphthalin beschränkt sich der Stand der Technik derzeit auf die Herstellung durch Azokupplung ausgehend von 1-Naphthylamin (JP-A2-51133237, Rev. Farm. Bioquim. Univ. Sao Paulo (1977), 15 (1-2), 19-25) und über die seit langem literaturbekannte Reaktion von Hydroxylamin mit Nitronaphthalin in stark alkalischem Medium zu 1-Nitro-4-aminonaphthalin (J. prakt. Chem. 156, 315 (1940), Chem. Ber., 22 (1899) 1374). Bei diesen Verfahren handelt es sich um vielstufige Synthesen mit hohem Produktionsaufwand und niedrigen Ausbeuten, die zusätzlich von kostenintensiven Einsatzstoffen ausgehen.

Polyurethane auf Basis von 1,4-Diisocyanatonaphthalin und 1,5-Diisocyanatonaphthalin besitzen vorteilhafte Eigenschaften, so dass ein praktikabler Syntheseweg zur Herstellung von 1,4-Diaminonaphthalin und 1,5-Diisocyanatonaphthalin erforderlich ist.

Die Aufgabe der vorliegenden Erfindung besteht darin, ein einfaches Verfahren für die Herstellung von 1,4-Diaminonaphthalin und/oder 1,5-Diaminonaphthalin zur Verfügung zu stellen.

Gegenstand der Erfindung ist ein Verfahren zur Herstellung von 1,4-Diaminonaphthalin und/oder 1,5-Diaminonaphthalin, enthaltend die Schritte
a) Umsetzung von Naphthalin mit Brom und/oder Chlor,
b) gegebenenfalls Abtrennung von 1,4-Dihalogennaphthalin oder 1,5-Dihalogennaphthalin aus dem Gemisch aus Schritt a),
c) Umsetzung des Gemisches aus Schritt a) oder des 1,4-Dihalogennaphthalins oder des 1,5-Dihalogennaphthalins aus Schritt b) mit Ammoniak und/oder einem organischen Amin in Gegenwart eines Katalysators.

Die selektive Dihalogenierung von Naphthalin kann unkatalysiert oder katalysiert, bevorzugt katalysiert, ablaufen.

Als Katalysatoren für die Dihalogenierung sind geeignet Iod, Zinn, Elemente der Gruppen 8, 10, 12 und 13 des Periodensystems (Nomenklatur nach I.U.P.A.C. 1985), mineralsaure Salze und Oxide der Elemente der Gruppen 8, 9, 10, 12, 13, 14 und 15 des Periodensystems (Nomenklatur nach I.U.P.A.C. 1985) sowie Mineralsäuren.

Bevorzugt werden Iod, Eisen, Zinn, Zink, Aluminium, Nickel, Halogenide oder Oxide von Eisen, Nickel, Aluminium, Antimon, Arsen, Zinn, Zink, Bor oder Phosphorsäure eingesetzt.

Als Lösemittel sind grundsätzlich alle Lösemittel geeignet, die Naphthalin zu lösen vermögen und nicht in nennenswertem Maße bei den herrschenden Bedingungen halogeniert werden. Bevorzugt werden Dichlormethan, Trichlormethan, Tribrommethan, Tetrachlormethan, Tetrabrommethan, Chlorbenzol, Dichlorbenzole und Trichlorbenzole, bevorzugt Trichlormethan, eingesetzt.

Bei der Dihalogenierung geht man vorzugsweise so vor, dass das Halogen, bevorzugt Brom, bei absoluten Drücken im Bereich von 0,5 bis 10 bar, bevorzugt von 1 bar bis 2 bar, besonders bevorzugt von 1 bar bis 1,2 bar, und bei Temperaturen im Bereich von 0°C bis 150°C, bevorzugt von 10°C bis 70°C, besonders bevorzugt von 20 bis 30°C, in eine Mischung aus Naphthalin, Lösemittel und Katalysator eingebracht wird. Die Naphthalinkonzentration bei der Halogenierung liegt vorzugsweise im Bereich von 5 bis 70 Gew.-%, besonders bevorzugt von 25 bis 50 Gew.-%, bezogen auf das Lösemittel.

Bei der Dihalogenierung entsteht ein Gemisch enthaltend 1,4-Dihalogennaphthalin und 1,5-Dihalogennaphthalin. Vor der Aminierung kann das Gemisch aufgereinigt werden. Es kann aber auch das 1,4-Dihalogennaphthalin oder das 1,5-Dihalogennaphthalin aus dem Gemisch abgetrennt werden. Auf diese Weise können das 1,4-Dihalogennaphthalin oder das 1,5-Dihalogennaphthalin isomerenrein aminiert werden.

Die Abtrennung des 1,4-Dihalogennaphthalins oder des 1,5-Dihalogennaphthalins aus dem Gemisch kann aufgrund der großen Schmelzpunktsdifferenz beispielsweise durch Kristallisation erfolgen.

Der Halogen-Amin-Austausch wird in Gegenwart eines Katalysators durchgeführt. Geeignete Katalysatoren sind Metalle der Gruppen 8, 9, 10 und 11 des Periodensystems (Nomenklatur nach I.U.P.A.C. 1985) und Oxide, Acetate und mineralsaure Salze von Metallen der Gruppen 8, 9, 10 und 11 des Periodensystems (Nomenklatur nach I.U.P.A.C. 1985).

Die Metalle können dabei beispielsweise in Form von Granalien, Spänen, Pulver oder trägerfixiert, beispielsweise auf Aluminium-, Silizium-, Titanoxid, oder in sonstiger fester Form eingesetzt werden. Die Chloride, Acetate und Oxide können beispielsweise in Form von Pellets oder als Pulver oder trägerfixiert, beispielsweise auf Aluminium-, Silizium-, Titanoxid, eingesetzt werden.

Bevorzugt werden Nickel-, Kupfer-, Cobalt- oder Eisen-Metall, Halogenide von Eisen, Nickel, Kupfer, Cobalt, Oxide von Eisen, Nickel, Kupfer, Cobalt, Acetate von Eisen, Nickel, Kupfer, Cobalt eingesetzt.

Als Lösemittel sind grundsätzlich alle Lösemittel geeignet, die Dihalogennaphthaline zu lösen vermögen und unter den herrschenden Bedingungen inert sind. Bevorzugt werden Wasser, Methanol, Ethanol, n-Propanol, n-Butanol, Acetonitril, Benzonitril, N,N-Dimethylacetamid, Dioxan oder Dioxan/Wasser-Gemische oder nicht mit Wasser mischbare inerte Lösemittel wie Toluol, Xylol oder aliphatische oder cycloaliphatische Kohlenwasserstoffe der C₆-C₁₂-Fraktion eingesetzt. Auch der Einsatz von flüssigem Ammoniak als Reaktionsmedium ohne weitere Lösemittelzugabe ist für die beschriebene Reaktion geeignet.

Als Aminierungsagens sind Ammoniak sowie primäre und sekundäre Amine, wie Monoalkylamine, Monoacylamine oder Dialkylamine geeignet. Bevorzugt wird Ammoniak eingesetzt.

Die Ausbeute der Halogen-Amin-Austauschreaktion bezüglich Diaminonaphthalin kann deutlich erhöht werden, wenn die bei der Reaktion entstehende Halogenwasserstoffsäure durch eine Base neutralisiert wird. Als Basen sind beispielsweise geeignet Carbonate und Hydroxide der Gruppen 1 und 2 des Periodensystems (Nomenklatur nach I.U.P.A.C. 1985). Bevorzugt wird Natrium-, Kalium-, Calcium oder Magnesiumcarbonat oder -hydrogencarbonat oder Natrium-, Kalium-, Calcium- oder Magnesiumhydroxid oder Gemische oder Mischkristalle daraus eingesetzt. Die Zugabe von Caesium-, Rubidium- oder Bariumchlorid, Caesium-, Rubidium- oder Bariumsulfat verbessert den ausbeutesteigernden Effekt der Base. Bevorzugt wird Caesiumchlorid oder Caesiumsulfat eingesetzt.

Der Halogen-Amin-Austausch wird bei Temperaturen im Bereich von 0°C bis 200°C, bevorzugt von 100 bis 180°C, besonders bevorzugt von 120 bis 160°C und bei Drücken von 1 bis 200 bar, bevorzugt 10 bis 150 bar, besonders bevorzugt bei 20 bis 120 durchgeführt. Die Konzentration an 1,4- und 1,5-Dihalogennaphthalin liegt vorzugsweise im Bereich von 5 bis 70 Gew.-%, besonders bevorzugt von 30 bis 50 Gew.-%, bezogen auf das Lösemittel bzw. auf Ammoniak im Falle einer lösemittelfreien Reaktion.

Nach der Aminierung kann ein Reinigungsschritt oder Trennschritt durchgeführt werden und das gewünschte Produkt aus dem Reaktionsgemisch entfernt werden. Geeignete Trennoperationen sind beispielsweise Destillation und Kristallisation.

Der Anteil des 1,5-Dihalogennaphthalins an dem Gemisch aus 1,4- Dihalogennaphthalin und 1,5-Dihalogennaphthalin liegt im Bereich von 1 und 50 Gew.-%, bevorzugt von 5 bis 40 Gew.-%, besonders bevorzugt von 10 bis 30 Gew.-%. Das Isomerenverhältnis wird durch die Halogenierung abhängig von der Katalysatorwahl und der Reaktionstemperatur bestimmt und bleibt bei der nachfolgenden Aminierung im Wesentlichen erhalten.

Gegenstand der Erfindung ist auch ein Verfahren zur Herstellung von 1,4-Diisocyanatonaphthalin und/oder 1,5-Diisocyanatonaphthalin, enthaltend die Schritte
a) Umsetzung von Naphthalin mit Brom und/oder Chlor,
b) Umsetzung des 1,4- und/oder 1,5-Dihalogennaphthalins aus Schritt a) mit Ammoniak in Gegenwart eines Katalysators, und
c) Phosgenierung des 1,4- und/oder 1,5-Diaminonaphthalins aus Schritt b).
wobei gegebenenfalls eine Abtrennung des 1,5 oder 1,4-Isomeren aus dem Isomerengemisch nach einem der Schritte a), b) oder c) erfolgt.

Die nach dem erfindungsgemäßen Verfahren hergestellten Amine können im Anschluss in an sich bekannter Weise zu den entsprechenden Isocyanaten phosgeniert und für die Herstellung von Polyurethanen eingesetzt werden (z.B. JP-A2-50012062). Nach der Phosgenierung kann ein Reinigungsschritt oder Trennschritt durchgeführt werden und das gewünschte Produkt, vorzugsweise 1,4-Diisocyanatonaphthalin, aus dem Reaktionsgemisch entfernt werden. Geeignete Trennoperationen sind beispielsweise Destillation und Kristallisation.

Die gegebenenfalls gewünschte Trennung des 1,4-Isomeren und des 1,5-Isomeren kann jedoch sowohl nach der Dihalogenierung, nach dem Halogen-Amin-Austausch oder nach der Phosgenierung erfolgen.

### Beispiele

### Beispiel 1

### Selektive Naphthalin-Bromierung in Tetrachlormethan

32 g Napthalin werden unter Rühren in einem 500 ml-Glaskolben zusammen mit 2,5 g Iod als Lösung in 170 ml Tetrachlormethan bei Raumtemperatur über einen Tropftrichter während 30 min mit 88 g Brom versetzt. Nach vollständiger Brom-Zugabe wird noch 1 h bei Raumtemperatur gerührt, anschließend wird durch Schütteln mit 100 ml 10 %iger wässriger Natriumsulfitlösung Bromüberschuss und Iod reduziert. Nach Abtrennen der wässrigen Phase wird die Produktlösung in Tetrachlormethan per Gaschromatograph analysiert.

Die ermittelte Selektivität bezüglich 1,4-/1,5-Diaminonaphthalin beträgt 97,25 % (GC). Das Verhältnis an 1,4-Diaminonaphthalin zu 1,5-Diaminonapthalin beträgt dabei 78,86 % : 18,39 % (GC).

Die zur Selektivitätsermittlung erforderlichen Analysen erfolgten mit einem Gaschromatographen (GC).

### Beispiel 2

### Selektive Naphthalin-Bromierung in Trichlormethan

128 g Napthalin werden unter Rühren in einem 2000 ml-Glaskolben zusammen mit 10 g Iod als Lösung in 700 ml Trichlormethan bei Raumtemperatur über einen Zeitraum von 1 h langsam mit 352 g Brom versetzt. Nach erfolgter Brom-Zugabe wird 1 h gerührt und anschließend das verbleibende Brom und Iod mit 500 ml einer 10 %igen wässrigen Natriumsulfitlösung ausgeschüttelt. Nach Abtrennen der wässrigen Phase und anschließendem Abdestillieren des Lösemittels verbleibt das Produkt.

Es werden 281,44 g Feststoff erhalten (= 98,4 mol % der Theorie, bezogen auf das eingesetzte Dibromnaphthalin). Die per Gaschromatograph ermittelte Reinheit des Produktes beträgt 97,63 % 1,4-/1,5-Diaminonaphthalin-Gemisch. Das Verhältnis 1,4-Diaminonaphthalin zu 1,5-Diaminonapthalin beträgt 85,34 % : 12,29 % (GC).

Die Gesamtausbeute an 1,4- und 1,5-Diaminonaphthalin-Gemisch wurde zu 96,1 % (GC) bestimmt.

### Beispiel 3

### a) Gemeinsame Abtrennung von Lösemittel und Iod-Katalysator (Lösemittel: Tetrachlormethan)

50 g eines 1,4-/1,5-Dibromnaphthalin-Gemisches hergestellt nach Beispiel 2 werden im Glaskolben mit 150 ml Tetrachlormethan und 1,1 g Iod versetzt. Bei einer Heißluftbeheizung mit 140°C wird das Lösemittel zusammen mit einem Großteil des Iods bei Normaldruck über eine Vigreux-Kolonne destillativ abgetrennt. Das verbleibende Jod wird mit 200°C Heißluft bei einem absoluten Druck von 10 mbar unter Einsatz von Stickstoff als Stripgas destillativ abgetrennt. Dabei ergibt sich nach beendeter Abtrennung ein farbloses Feststoffprodukt.

### b) Gemeinsame Abtrennung von Lösemittel und Iod-Katalysator (Lösemittel: Tribrommethan)

50 g eines 1,4-/1,5-Dibromnaphthalin-Gemisches hergestellt nach Beispiel 2 werden im Glaskolben mit 150 ml Tetrabrommethan und 1,1 g Iod versetzt. Bei einem absoluten Druck von 50 mbar und einer Sumpftemperatur von 100°C wird das Lösemittel zusammen mit dem Iod destillativ abgetrennt. Dabei ergibt sich nach beendeter Abtrennung des Lösemittels bereits ein farbloses Feststoffprodukt.

### Beispiel 4

### Brom-/Amino-Austausch an 1,4-/1,5-Dibromnaphthalin-Gemisch mit CuCl₂ (wasserfrei)

50 g eines 1,4-/1,5-Dibromnaphthalin-Gemisches hergestellt nach Beispiel 2 werden zusammen mit 10 g CuCl₂ (wasserfrei) und 250 ml 25 %igem Ammoniakwasser in einen 500 ml-Druckautoklaven gegeben. Es werden zusätzlich 100 g Ammoniak aufgepresst und anschließend auf 150°C erwärmt, wobei sich der Druck auf 38 bar erhöht.

Nach 6 h Reaktionszeit wird die Reaktion durch Abkühlen und Abblasen des verbleibenden Ammoniaks beendet. Die per Gaschromatographie ermittelte Selektivität der Reaktion bezüglich des 1,4-/1,5-Diaminonaphthalin-Gemisches lag bei 61,50 % (GC).

### Beispiel 5

### Brom-/Amino-Austausch an 1,4-/1,5-Dibromnaphthalin-Gemisch mit CuCl (wasserfrei)

40 g eines 1,4-/1,5-Dibromnaphthalin-Gemisch hergestellt nach Beispiel 2 werden zusammen mit 6 g CuCl (wasserfrei) und 250 ml 25%igem Ammoniakwasser in einen 500 ml-Druckautoklaven gegeben. Es werden zusätzlich 100 g Ammoniak aufgepresst und anschließend der Reaktorinhalt unter Rühren auf 150°C erwärmt, wobei sich der Druck auf 41 bar erhöht.

Nach 6 h Reaktionszeit wird die Reaktion durch Abkühlen und Abblasen des verbleibenden Ammoniaks beendet. Die per Gaschromatographie ermittelte Selektivität der Reaktion bezüglich des 1,4-/1,5-Diaminonaphthalingemisches lag bei 61,32 % (GC).

### Beispiel 6

### Phosgenierung von 1,4-Diaminonaphthalin zum 1,4-Diisocyanatonaphthalin

Aus einer Lösung von 250 g 1,4-NDA in 2 1 96 %igem Ethanol werden mit 300 ml 37 %iger Salzsäure versetzt. Das ausgefallene 1,4-Diaminonaphthalindihydrochlorid wird abfiltriert, mit wenig 96 %igem Ethanol gewaschen und getrocknet. Es werden 323 g 1,4-Diaminonaphthalindihydrochlorid erhalten.

188,9 g 1,4-Diaminonaphthalindihydrochlorid mit 99,3 % Reinheit werden in einem 10 1-Glaskolben in 6720 ml Monochlorbenzol suspendiert. Nach azeotroper Entwässerung unter Abdestillieren von 400 ml Monochlorbenzol wird unter Rühren bei einer Temperatur von 80°C 1 h über ein Glasrohr Phosgen mit einer Menge von 260 g/h eingeleitet. Anschließend wird bei einer Temperatur von 100°C bei gleicher Phosgenzugabe-Menge ca. 12 h unter Rühren nachphosgeniert, wobei sich die zunächst erhaltene Suspension klärt.

Nach 12 h Entphosgenierung durch Einblasen von Stickstoff unter Rühren bei Raumtemperatur wird das Monochlorbenzol destillativ abgetrennt und die Rohware bei einer Heißlufttemperatur von ca. 260°C und einem Vakuum < 0,1 mbar vordetilliert. Die Rohdestillatmenge beträgt 156 g (= 90,9 % der Theorie, bezogen auf das eingesetzte 1,4-Diaminonaphthalin).

Nach Feindestillation des Rohdestillates über einer 50 cm-Vigreux-Kolonne werden 141 g 1,4-Diisocyanatonaphthalin mit einer Gaschromatograph-Reinheit von 99,5 % (GC) erhalten.

### Beispiel 7

### Brom-/Amino-Austausch an 1,4-/1,5-Dibromnaphthalin-Gemisch mit Kupferacetat unter Zusatz einer zusätzlichen Base

30 g eines 1,4-/1,5-Dibromnaphthalin-Gemisches hergestellt nach Beispiel 2 werden zusammen mit 9 g Cu(CH₃COO)₂*H₂O, 14,5 g K₂CO₃, 3,6 g CsCl und 150 ml 25 %igem Ammoniakwasser in einen 500 ml-Druckautoklaven gegeben. Es werden zusätzlich 150 g Ammoniak aufgepresst und anschließend der Reaktorinhalt unter Rühren auf 120°C erwärmt, wobei sich der Druck auf 39 bar erhöht.

Nach 20 h Reaktionszeit wird die Reaktion durch Abkühlen und Abblasen des verbleibenden Ammoniaks beendet. Die per Gaschromatographie ermittelte Selektivität der Reaktion bezüglich des 1,4-/1,5-Diaminonaphthalingemisches lag bei 79,48 % (GC).

## Patentansprüche

1. Verfahren zur Herstellung von 1,4-Diaminonaphthalin und/oder 1,5-Diaminonaphthalin, enthaltend die Schritte
a) Umsetzung von Naphthalin mit Brom und/oder Chlor,
b) gegebenenfalls Abtrennung von 1,4-Dihalogennaphthalin oder 1,5 Dihalogennaphthalin aus dem Gemisch aus Schritt a),
c) Umsetzung des Gemisches aus Schritt a) oder des 1,4-Dihalogennaphthalins oder des 1,5-Dihalogennaphthalins aus Schritt b) mit Ammoniak in Gegenwart eines Katalysators.

2. Verfahren nach Anspruch 1, bei dem die Dihalogenierung in Gegenwart eines Katalysators durchgeführt wird ausgewählt aus der Gruppe bestehend aus Iod, Zinn, Elementen der Gruppen 8, 10, 12 und 13 des Periodensystems, mineralsauren Salzen und Oxiden der Elemente der Gruppen 8, 9, 10, 12, 13, 14 und 15 des Periodensystems sowie Mineralsäuren.

3. Verfahren nach Anspruch 2, bei dem als Katalysator Eisen-, Zink-, Aluminium-, Nickel-Metall, Halogenide oder Oxiden von Eisen, Nickel, Aluminium, Antimon, Arsen, Zinn, Zink oder Bor oder konzentrierte Phosphorsäure eingesetzt werden.

4. Verfahren nach einem der Ansprüche 1 bis 3, bei dem man den Halogen-Amin-Austausch in Gegenwart eines Katalysators durchführt ausgewählt aus der Gruppe bestehend aus Metallen der Gruppen 8, 9, 10 und 11 des Periodensystems und Oxiden, Acetaten und mineralsauren Salzen von Metallen der Gruppen 8, 9, 10 und 11 des Periodensystems.

5. Verfahren nach Anspruch 4, bei dem als Katalysator Nickel-, Kupfer-, Cobalt- oder Eisen-Metall oder Halogenide oder Oxide von Eisen, Nickel, Kupfer oder Cobalt einsetzt werden.

6. Verfahren nach Anspruch 4, bei dem man den Halogen-Amin-Austausch zusätzlich in Gegenwart einer Base durchführt.

7. Verfahren zur Herstellung von 1,4-Diisocyanatonaphthalin und/oder 1,5-Diisocyanatonaphthalin, enthaltend die Schritte
a) Umsetzung von Naphthalin mit Brom und/oder Chlor,
b) Umsetzung des 1,4- und/oder 1,5-Dihalogennaphthalins aus Schritt a) mit Ammoniak in Gegenwart eines Katalysators, und
c) Phosgenierung des 1,4- und/oder 1,5-Diaminonaphthalins aus Schritt b),
wobei gegebenenfalls eine Abtrennung des 1,5- oder 1,4-Isomeren aus dem Isomerengemisch nach einem der Schritte a), b) oder c) erfolgt.

## Claims

1. Process for preparing 1,4-diaminonaphthalene and/or 1,5-diamino-naphthalene, containing the steps
a) reacting of naphthalene with bromine and/or chlorine,
b) optionally separation of 1,4-dihalogen naphthalene or 1,5-dihalogen naphthalene from the mixture from step a),
c) reacting of the mixture from step a) or of the 1,4-dihalogen naphthalene or of the 1,5-dihalogen naphthalene from step b) with ammonia in the presence of a catalyst.

2. Process according to claim 1, in which the dihalogenation is carried out in the presence of a catalyst selected from the group consisting of iodine, tin, elements of Groups 8, 10, 12 and 13 of the Periodic Table, mineral acid salts and oxides of elements of Groups 8, 9, 10, 12, 13, 14 and 15 of the Periodic Table and mineral acids.

3. Process according to claim 2, in which there are used as catalyst iron, zinc, aluminium or nickel metal, halides or oxides of iron, nickel, aluminium, antimony, arsenic, tin, zinc or boron or concentrated phosphoric acid.

4. Process according to one of claims 1 to 3, in which the halogen-amine substitution is performed in the presence of a catalyst selected from the group consisting of metals of Groups 8, 9, 10 and 11 of the Periodic Table and oxides, acetates and mineral acid salts of metals of Groups 8, 9, 10 and 11 of the Periodic Table.

5. Process according to claim 4, in which there are used as catalyst nickel, copper, cobalt or iron metal or halides or oxides of iron, nickel, copper or cobalt.

6. Process according to claim 4, in which the halogen-amine substitution is carried out additionally in the presence of a base.

7. Process for preparing 1,4-diisocyanatonaphthalene and/or 1,5-diisocyanatonaphthalene, containing the steps
a) reacting of naphthalene with bromine and/or chlorine,
b) reacting of the 1,4- and/or 1,5-dihalogen naphthalene from step a) with ammonia in the presence of a catalyst, and
c) phosgenation of the 1,4- and/or 1,5-diaminonaphthalene from step b).
wherein optionally a separation of the 1,5 or 1,4 isomer from the isomer mixture takes place after one of steps a), b) or c).

## Revendications

1. Procédé pour la préparation du 1,4-diaminonaphtalène et/ou du 1,5-diaminonaphtalène, comprenant les stades opératoires suivants :
a) réaction du naphtalène avec le brome et/ou le chlore,
b) le cas échéant séparation du 1,4-dihalogénonaphtalène ou du 1,5-dihalogénonaphtalène à partir du mélange obtenu au stade opératoire a),
c) réaction du mélange obtenu au stade opératoire a) ou du 1,4-dihalogénonaphtalène ou du 1,5-dihalogénonaphtalène obtenu au stade opératoire b) avec l'ammoniac en présence d'un catalyseur.

2. Procédé selon la revendication 1 dans lequel la dihalogénation est réalisée en présence d'un catalyseur choisi dans le groupe consistant à l'iode, l'étain, les éléments des groupes 8, 10, 12 et 13 de la Classification Périodique, les sels minéraux et oxydes des éléments des groupes 8, 9, 10, 12, 13, 14 et 15 de la Classification Périodique et les acides minéraux.

3. Procédé selon la revendication 2, dans lequel on utilise en tant que catalyseur le fer, le zinc, l'aluminium, le nickel métalliques, un halogénure ou oxyde du fer, du nickel, de l'aluminium, de l'antimoine, de l'arsenic, de l'étain, du zinc ou du bore ou l'acide phosphorique concentré.

4. Procédé selon l'une des revendications 1 à 3, dans lequel l'échange halogène-amine est réalisé en présence d'un catalyseur choisi dans le groupe consistant en les métaux des groupes 8, 9, 10 et 11 de la Classification Périodique et les oxydes, acétates et sels minéraux des métaux des groupes 8, 9, 10 et 11 de la Classification Périodique.

5. Procédé selon la revendication 4, dans lequel on utilise en tant que catalyseur le nickel, le cuivre, le cobalt ou le fer métalliques ou un halogénure ou oxyde du fer, du nickel, du cuivre ou du cobalt.

6. Procédé selon la revendication 4, dans lequel l'échange halogène-amine est réalisé en outre en présence d'une base.

7. Procédé pour la préparation du 1,4-diisocyanatonaphtalène et/ou du 1,5-diisocyanatonaphtalène, comprenant les stades opératoires suivants
a) réaction du naphtalène avec le brome et/ou le chlore,
b) réaction du 1,4- et/ou du 1,5-dihalogénonaphtalène obtenu au stade opératoire a) avec l'ammoniac en présence d'un catalyseur et
c) phosgénation du 1,4- et/ou du 1,5-diaminonaphtalène obtenu au stade opératoire b),
avec le cas échéant séparation de l'isomère 1,5 ou de l'isomère 1,4 à partir du mélange d'isomères, après exécution de l'un des stades opératoires a), b) ou c).
